# EUROPEAN PATENT SPECIFICATION

(11) **EP 0 344 708 B1**
(45) Date of publication and mention of the grant of the patent: **28.04.1993**
(21) Application number: 89109723.0
(22) Date of filing: 30.05.1989
(51) Int. Cl.: A61K 7/48, A61K 31/70

(54) **Composition for external application to skin**
Präparat zum äusserlichen Gebrauch auf der Haut
Composition pour l'application externe sur la peau

(30) Priority: 31.05.1988 JP 133810/88
(43) Date of publication of application: 06.12.1989
(73) Proprietor: LION CORPORATION, Sumida-ku Tokyo (JP)
(72) Inventor: Sugiyama, Keikichi, Naka-gun Kanagawa-ken (JP); Egawa, Makoto, Odawara-shi Kanagawa-ken (JP); Takeuchi, Keiji, Setagaya-ku Tokyo (JP)
(74) Representative: KUHNEN, WACKER & PARTNER

(56) References cited:
- EP-A- 0 256 472
- DE-A- 2 459 932
- CHEMICAL ABSTRACTS, vol. 83, no. 8, 25th August 1975, page 436, abstract no. 65339a, Columbus, Ohio, US

## Description

### BACKGROUND OF THE INVENTION

### Field of the Invention

The present invention relates to compositions for external application to the skin such as cosmetic compositions as well as an agents for treating traumas, for instance, cosmetic creams and ointments, which have a variety of effects such as activation of dermal cells, promotion of their metabolism and healing of traumas and which, upon application to the skin, prevent aging of the skin and make the skin wrinkle-free, smooth, moist and young-looking.

### Prior Art

Although animal and plant extracts, vitamins, amino acids and nucleic acids which are extracted from natural substances have been conventionally incorporated into compositions for application to the skin such as cosmetics, for the purpose of preventing aging of the skin, no marked effect concerning the prevention of aging can be expected, even if a relatively large amount of them is incorporated into such compositions. This is because these components show only auxiliary functions.

It has also been proposed that prostaglandins which are physiologically active substances present in small amounts in organisms (see Japanese Patent Un-examined Publication (hereinafter referred to as "J.P. KOKAI") No. Sho 48-18436); coenzyme A (see J.P. KOKAI No. Sho 50-31051 and Chemical Abstracts vol 83, no. 8, abstract no. 65339a); and epidermal growth factor (EGF) or urogastrone (see J.P.KOKAI No. Sho 61-5006) could be utilized in cosmetics. However, it is found that they show only insufficient effects and their effective components lack stability.

For instance, the coenzyme A and salts thereof are unstable in preparations. Therefore, if they are incorporated into compositions such as cosmetics, they exert even adverse effects on other components. For instance, they cause deterioration of the color tone of other components.

### SUMMARY OF THE INVENTION

Accordingly, an object of the present invention is to provide a composition for external application to the skin which shows marked effects concerning the prevention of aging of the skin and trauma healing effects and which is stable in various preparations and is of much practical use.

The foregoing and other objects of the present invention will be apparent from the following detailed description.

The present invention has been achieved on the basis of the knowledge that the aforementioned object of the present invention can effectively be attained by providing a composition for external application to skin which comprises 0.001 to 5 % by weight of CoA-disulfide represented by the following formula (I) and/or a salt thereof selected from the group consisting of alkali metal, alkaline earth metal and zinc family metal salts of the phosphate residue of Compound (I): 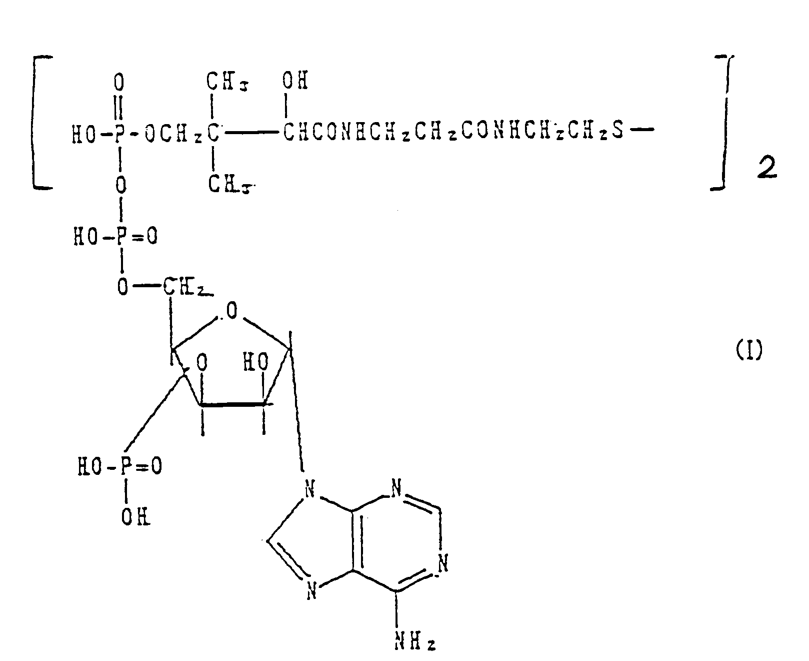
0 to 80% by weight of an oil component, 0 to 12% by weight of a surfactant, 1 to 15% by weight of a humectant, a small amount of a preservative and the balance of an inert carrier.

According to other aspects of the present invention, there are provided cosmetic compositions for application to the skin and a composition for healing traumas which comprise CoA-disulfide represented by the formula (I) and salts thereof.

### DETAILED EXPLANATION OF THE INVENTION

The present invention will hereinafter be described in more detail.

The composition for external application to the skin according to the present invention can comprise one or more inert carriers such as water, ethanol and isopropanol.

Compound (I) used in the invention can be obtained by oxidizing coenzyme A, which can be prepared by a fermentation technique or can be extracted from animal organs, to dehydrogenate and form an SS bond. Therefore, Compound (I) is also referred to as oxidized coenzyme A or coenzyme A oxidized. The resultant Compound (I) shows high stability in a variety of compositions.

In the invention, metal salts of the phosphate residue of Compound (I) can be used and examples of such salts are alkali metal salts such as sodium, potassium and lithium salts; alkaline earth metal salts such as calcium and magnesium salts; and salts derived from zinc family metals such as zinc salt. When the salts of Compound (I) are divalent metal salts, such a salt is formed from an adjacent pair of phosphate residues. More specifically, examples of such salts are disodium salts (hereinafter referred to as "I ·2Na"; those in the following description are shown in the same way also), hexasodium salts (I ·6Na), dipotassium salts (I ·2K), hexapotassium salts ((I ·6K), dicalcium salts (I ·2Ca), and dizinc salts (I ·2Zn) of Compound (I).

In the present invention, Compound (I) and salts thereof can be used alone or in combination.

The composition for external application to the skin of the invention shows excellent effects concerning the prevention of aging of the skin which have not been conventionally known, is stable over a long time period and is hence of much practical use because of the use of Compound (I) and/or salts thereof as essential components. The essential components may be incorporated into the composition for external application to the skin in any concentration. In general, these essential components are added to the composition for external application to the skin in an amount ranging from 0.001 to 5% by weight (hereinafter referred to as simply "%"), preferably 0.01 to 2% on the basis of the total weight of the composition.

In addition to the foregoing components, the composition of the invention may further comprise various ingredients commonly used in agents externally applied such as surfactants, oil components, alcohols, humectants, thickening agents, preservatives, antioxidants, chelating agents, pH adjusting agents, perfumes, color additives, ultraviolet absorbing, scattering agents, vitamins, amino acids and/or water.

Specific examples of the surfactants usable in the invention are nonionic surfactants such as lipophilic glyceryl monostearate, self-emulsifying glyceryl monostearate, polyglyceryl monostearate, sorbitan monooleate, polyethyleneglycol monostearate, polyoxyethylene sorbitan monooleate, polyoxyethylene cetylether, polyoxyethylene sterol, polyoxyethylene lanolin, polyoxyethylene beeswax and polyoxyethylene hydrogenated castor oil; anionic surfactants such as sodium stearate, potassium palmitate, sodium cetyl sulfate, sodium lauryl phosphate, triethanolamine palmitate sodium polyoxyethylene lauryl phosphate and sodium N-acylglutamate; cationic surfactants such as stearyldimethylbenzylammonium chloride and stearyltrimethylammonium chloride; and amphoteric surfactants such as alkylaminoethylglycine hydrochloride solution and lecithin.

Specific examples of the oil components are plant fats and oils such as castor oil, olive oil, cacao butter, tsubaki oil, coconut oil, Japan wax, jojoba oil, grapeseed oil, avocado oil; animal fats and oils such as egg yolk oil; waxes such as beeswax, spermaceti, lanolin, carnauba wax and candelilla wax; hydrocarbons such as liquid paraffin, squalane, microcrystalline wax, ceresin wax, paraffin wax, vaseline; natural and synthetic fatty acids such as lauric acid, myristic acid, stearic acid, oleic acid, isostearic acid and behenic acid; natural and synthetic higher alcohols such as cetanol, stearyl alcohol, hexyldecanol, octyldodecanol and lauryl alcohol; and esters such as isopropyl myristate, isopropyl palmitate, octyldodecyl myristate, octyldodecyl oleate and cholesterol oleate.

Examples of the humectants usable in the invention include polyhydric alcohols such as glycerin, propylene glycol, 1,3-butylene glycol, sorbitol, polyglycerin, polyethylene glycol and dipropylene glycol; NMF components such as amino acids, sodium lactate, sodium pyrrolidone carboxylate; and water-soluble polymeric substances such as hyaluronic acid, collagen, monopolysaccharide and chondroitin sulfate.

Examples of the thickening agents usable in the invention include sodium alginate, xanthane gum, aluminum silicate, extract of quince seeds, tragacanth gum and starches; semisynthetic polymeric substances such as methyl cellulose, hydroxyethyl cellulose, carboxymethyl cellulose, soluble starches and cationized cellulose; and synthetic polymeric substances such as carboxyvinyl polymers and polyvinyl alcohol.

Examples of the preservatives usable in the invention are benzoic acid salts, salicylic acid salts, sorbic acid salts, dehydroacetic acid salts, paraoxybenzoic acid esters, 2,4,4′-trichloro-2′-hydroxydiphenylether, 3,4,4′-trichlorocarbanilide, benzalkonium chloride, hinokitiol, resorcin and ethanol.

Examples of the antioxidants usable in the invention include dibutylhydroxytoluene, butylhydroxyanisole, propyl gallate and ascorbic acid; examples of the chelating agents are ethylenediaminetetraacetic acid salts, pyrophosphoric acid salts, hexametaphosphoric acid salts, citric acid, tartaric acid and gluconic acid; and examples of the pH adjusting agents are sodium hydroxide, triethanolamine, citric acid, sodium citrate, boric acid, borax and potassium hydrogen phosphate.

As the ultraviolet absorbing and scattering agents, there may be named 2-hydroxy-4-methoxybenzophenone, octyldimethyl p-aminobenzoate, ethylhexyl p-methoxycinnamate, titanium oxide, kaolin and talc.

Examples of the vitamins usable in the invention are vitamin A, vitamin B, vitamin C, vitamin D, vitamin E, vitamin F, vitamin K, vitamin P, vitamin U, carnitine, ferulic acid, γ-oryzanol, α-lipoic acid, orotic acid and derivatives thereof.

Examples of the amino acids as used herein are glycine, alanine, valine, leucine, isoleucine, serine, threonine, phenylalanine, tyrosine, tryptophan, cystine, cysteine, methionine, proline, hydroxyproline, aspartic acid, glutamic acid, arginine, histidine, lysine and derivatives thereof.

In the invention, optional components are not restricted to these specific compounds listed above. The composition for external application to the skin of this invention can be prepared by appropriately mixing the foregoing essential components, inert carriers and optional components. The composition comprises 0.001 to 5% of the essential components, 0 to 80% of oil components, 0 to 12% of surfactants, 1 to 15% of humectants, a small amount of preservatives and the balance of inert carriers such as purified water. The composition of the invention may be used in any form such as creams, milky lotions, toilet waters, beauty essences, packs, undermakeups, foundations, jellies, and ointmemts.

When the composition is used as a cosmetic composition for application to the skin, for instance:
Creams may comprise 0.01 to 2% of the essential components, 20 to 70% of oil components, 2 to 7% of surfactants, 1 to 10% of humectants, small amounts of preservatives and perfumes and the balance of purified water;
Milky lotions may comprise 0.01 to 2% of the essential components, 10 to 40% of oil components, 0 to 15% of alcohols, 1 to 5% of surfactants, 1 to 10% of humectants, 0 to 2% of thickening agents, small amounts of preservatives and perfumes and the balance of purified water;
Toilet waters and beauty essences may comprise 0.01 to 2% of the essential components, 5 to 20% of alcohols, 0 to 2% of surfactants, 2 to 8% of humectants, 0 to 2% of thickening agents, 0 to 0.5% of antioxidants, 0 to 0.1% of chelating agents, 0 to 0.2% of pH adjusting agents, small amounts of preservatives and perfumes, 0 to a small amount of color additives and the balance of purified water;
Packs may comprise 0.01 to 2% of the essential components, 2 to 10% of alcohols, 2 to 10% of humectants, 0 to 20% of inorganic powder, 10 to 20% of film-forming agents, small amounts of preservatives and perfumes and the balance of purified water.

When the composition of the invention is used as an agent for healing or treating traumas, for instance, ointments (hydrophilic ointments) may comprise 0.01 to 2% of the essential components, 40 to 60% of oil components, 1 to 12% of surfactants, 8 to 15% of humectants, a small amount of preservatives and the balance of purified water.

Ointments (oleophilic ointments) may comprise 0.01 to 2% of the essential components, 95 to 99% of oil components and the balance of purified water.

Although it is not known in detail how the composition for external application to the skin according to the present invention exhibits an excellent function of preventing the aging of the skin, it is assumed that Compound (I) or salts thereof as the essential components of the invention is absorbed through the skin to activate dermal fibroblasts and, as a result, the components improve elasticity and softness of the skin and hence provide excellent effect of preventing the aging of the skin. Moreover, it is also confirmed that the components show very high trauma healing effects.

In addition, these essential components are quite stable during manufacturing the preparations as the composition for external application to the skin and even during storage and thus are of much practical use.

The safety of the foregoing effective components used in the invention was examined and it was found that they had not any practical particular problem concerning, for instance, acute toxicity, skin irritation and skin sensitization. Thus, it was confirmed that they show very high safety.

The composition for external application to the skin according to the present invention activates dermal cells and prevents the aging of the skin. In other words, the composition shows marked effects that they prevent the generation of wrinkles and/or spots on the skin and provide smooth, moist and young-looking skin and that they markedly enhance the regeneration-promoting effect of injured parts. Moreover, they provide compositions for external application to the skin which are quite stable from the viewpoint of manufacture of preparations.

Therefore, the compositions for external application to the skin according to the present invention can be widely used in a variety of forms such as cosmetic creams, milky lotions, toilet waters, beauty essences, packs, undermakeups, foundations, jellys and ointments.

The present invention will hereunder be explained in more detail with reference to the following non-limitative working Examples and the effects practically achieved by the invention will also be discussed in comparison with Comparative Examples.

### Example 1

The components 1 to 7 and the components 8 to 13 listed in Table I were separately mixed and dissolved. To the solution of the components 8 to 13 there was added the solution of the components 1 to 7 with stirring to emulsify these components. Then the emulsified mixture was cooled to room temperature. The component 14 was added to the emulsified mixture in the course of cooling to obtain a cream shown in Table I. In Table I, each numerical value represents the amount of each component incorporated (expressed in "% by weight"; those in the following Tables are shown in the same way also).

**Table I**

| Component | Present Inv. | Comp. Example |
|---|---|---|
| 1. Liquid paraffin (#70) | 5.0 | 5.0 |
| 2. Squalane | 15.0 | 15.0 |
| 3. Cetostearyl alcohol | 5.0 | 5.0 |
| 4. Beeswax | 2.0 | 2.0 |
| 5. Glyceryl monostearate | 2.0 | 2.0 |
| 6. Polyoxyethylene (POE: 20) sorbitan monolaurate | 2.0 | 2.0 |
| 7. Propylparaben | 0.1 | 0.1 |
| 8. Compound I ·6Na | 0.5 | -- |
| 9. Coenzyme A ·3Na | -- | 0.5 |
| 10. Hyaluronic acid | 0.2 | 0.2 |
| 11. Diglycerin | 5.0 | 5.0 |
| 12. Methylparaben | 0.2 | 0.2 |
| 13. Purified water | balance | balance |
| 14. Perfume | small amount | small amount |

The stability of the effective components (components 8 and 9) in the cream compositions thus prepared, during storage, was examined by a high performance liquid chromatography analysis (HPLC analysis) and visual evaluation of its color change. The results obtained are listed in Table II given below.

**Table II**

| Items Evaluated | Present Inv. | Comp. Example | Conditions |
|---|---|---|---|
| Remaining effective component(%) | 95 | no abs. peak of Component 9 | After storage at 25°C for one month |
| Color of cream | no particular change | changed to pink or brown | After storage at 45 °C for one month |

As seen from the results listed in Table II, it was also found that the effective component (I ·6Na) of the present invention was quite stably present in the composition and that no color change of the cream composition was observed.

### Example 2

Groups of Wistar male rats (SPF; each group comprising 8 animals) were anesthetized with Nembutal® (pentobarbital sodium) injection and hair was removed from the back of each rat over a wide area. After disinfecting the area with alcohol, a sharp incised wound having a length of 4 cm was mesially formed with a scalpel and it was stitched up, with Mitchell needles, at three positions at regular intervals (the Mitchell needles were removed 4 days after the operation).

After forming incised wound, an effective component was dissolved in a saline solution and administered subcutaneously once a day for 7 days successively (dose: 0.67 m ℓ/kg/day). The animals were sacrificed 8 days after the operation, the skin of the wounded part was peeled off to remove subcutis followed by preparing three pieces of the skin perpendicular to the line of the incised wound, having a width of 1 cm, per rat and determining tention (g/cm) required for peeling off each piece of the skin at the incised wound portion utilizing a tensile strength tester. The relative value of the average of the measured tension with respect to that of the control was defined as an indication of the rate of healing of the wound. The results obtained are listed in Table III given below (the results are expressed in the value relative to that of the control which is defined as 100).

**Table III**

| Medical Substance | Concn.(%) | Rate of Relative Healing |
|---|---|---|
| Present Inv. (Compound I ·6Na) | 0.5 | 126 |
| Comp. Ex. (Coenzyme A ·3Na) | 0.5 | 117 |
| Control (Physiological Saline) | -- | 100 |

Compound (I ·6Na) used in the present invention showed a remarkable wound healing effect. This clearly indicates that the healing of the wound is promoted.

### Example 3

Groups of Wistar male rats (SPF; each group comprising 6 animals) were anesthetized and hair was removed from the back of each rat. Then a circular defective wound having a diameter of 12 mm was formed on the skin of the back from which hair had been removed.

After forming the defective wound, an effective component was dissolved in 10% ethanol aqueous solution and applied to the wounded portion twice a day in a dose of 1 mℓ per application for 5 days successively. 6 Days after the formation of the wound, the animals were sacrificed to cut off the formed granulation from the defective portion and the weight of the granulation was determined. The relative value of the average of the measured weight with respect to that of the control was defined as an indication of the rate of healing of the defective wound. The results obtained are listed in Table IV given below (the results are expressed in the value relative to that of the control which is defined as 100).

**Table IV**

| Medical Substance | Concn.(%) | Rate of Regenerated Granulation |
|---|---|---|
| Present Inv. (Compound I · 6Na) | 0.1 | 148 |
| Comp. Ex. (Coenzyme A ·3Na) | 0.1 | 119 |
| Control (10% ethanol aqueous solution) | -- | 100 |

As seen from the results listed in Table IV, Compound (I·6Na) used in the present invention shows excellent granulation regeneration effect. This is resulted from the promotion of proliferation of fibroblasts at the wounded part due to Compound I ·6Na.

As discussed above in detail, the compounds used in the present invention as essential components show excellent wound healing effects.

### Example 4

To the component 7 there were added successively the components 1 to 6 shown in Table V to dissolve these components and further the component 8 was added to the solution to prepare a toilet water shown in Table V.

**Table V**

| Component | Present Inv. | Comp. Ex. |
|---|---|---|
| 1. Compound I | 0.1 | -- |
| 2. 1,3-Butylene glycol | 3.0 | 3.0 |
| 3. Citric acid | 0.02 | 0.02 |
| 4. Sodium citrate | 0.05 | 0.05 |
| 5. Ethanol | 15.0 | 15.0 |
| 6. Methylparaben | 0.1 | 0.1 |
| 7. Purified water | balance | balance |
| 8. Perfume | small amount | small amount |

The effectiveness of the toilet water thus prepared was evaluated as follows:

20 Statistically equivalent women (35 to 55-year-old) were selected and the toilet waters of the present invention and Comparative Example were applied separately to the right and left face portions, respectively, of each woman by a half face method twice a day (in the morning and at night) for 3 months successively. The degree of improvement in terms of fine-wrinkling of the skin and glossiness and fineness of the skin were then examined. The results obtained are summarized in Table VI given below.

**Table VI**

| Items Evaluated | | Present Inv. | Comp. Ex. |
|---|---|---|---|
| Improvement of Fine-wrinkles | effective | 4 | 0 |
| | effective in some degree | 12 | 0 |
| | ineffective | 4 | 20 |
| Improvement of Fineness and Glossiness of the Skin | effective | 5 | 0 |
| | effective in some degree | 12 | 1 |
| | ineffective | 3 | 19 |

As seen from the results listed in Table VI, the toilet water of the present invention into which Compound I was incorporated showed marked effects of improving fine-wrinkles and glossiness and fineness of the skin compared with that of Comparative Example free of the compound. In addition, during and after the use of the toilet water for 3 months, any abnormality such as rubefaction was not observed in the condition of the skin.

Moreover, after the completion of the test, the stability of Compound I in the used toilet water of the present invention was examined by high performance liquid chromatography (HPLC) and it was found that the compound showed high stability since the rate of the remaining Compound I was 97.8% on the average.

### Example 5

The components 1 to 4 and the components 5 to 9 shown in Table VII below were separately dissolved and then these two solutions were admixed together to obtain a beauty essence.

**Table VII**

| Component | Present Inv. | Comp. Ex. |
|---|---|---|
| 1. Compound I ·6Na | 0.1 | -- |
| 2. Glycerin | 4.0 | 4.0 |
| 3. Carboxyvinyl polymer | 0.5 | 0.5 |
| 4. Purified water | balance | balance |
| 5. dl-α-Tocopheryl acetate | 0.2 | 0.2 |
| 6. Ethanol | 10.0 | 10.0 |
| 7. POE (40) hydrogenated castor oil | 0.5 | 0.5 |
| 8. Methylparaben | 0.1 | 0.1 |
| 9. Perfume | small amount | small amount |

The effectiveness of these beauty essences was examined according in the same manner as in Example 4 and the results obtained were listed in the following Table VIII.

**Table VIII**

| Items Evaluated | | Present Inv. | Comp. Ex. |
|---|---|---|---|
| Improvement of Fine-wrinkles | effective | 7 | 0 |
| | effective in some degree | 11 | 1 |
| | ineffective | 2 | 19 |
| Improvement of Fineness and Glossiness of the Skin | effective | 8 | 0 |
| | effective in some degree | 11 | 2 |
| | ineffective | 1 | 18 |

As seen from the results listed in Table VIII, the beauty essence of the present invention into which Compound I ·6Na was incorporated showed marked effects of improving fine-wrinkles and glossiness and fineness of the skin compared with that of Comparative Example free of the compound. In addition, during and after the use of the beauty essence for 3 months, any abnormality such as rubefaction was not observed in the condition of the skin.

Moreover, after the completion of the test, the stability of Compound I ·6Na in the used beauty essence of the present invention was examined by HPLC and it was found that the compound showed high stability since the rate of Compound I ·6Na remaining in the beauty essence was 98.5% on the average.

### Example 6

The components 1 to 7 shown in Table IX were dissolved by heating them at 70° C. Separately, the components 8 to 13 were dissolved by heating these at 70 ° C, the foregoing solution for fats and oils (the components 1 to 7) was added to the resulting solution of the components 8 to 13 to emulsify these. Then, the emulsified mixture was cooled to room temperature and the component 14 was added to the emulsion in the course of the cooling to obtain a milky lotion as shown in Table IX.

**Table IX**

| Component | Present Inv. | Comp. Ex. |
|---|---|---|
| 1. Liquid paraffin (#70) | 10.0 | 10.0 |
| 2. Isopropyl myristate | 2.0 | 2.0 |
| 3. Glyceryl monostearate | 0.5 | 0.5 |
| 4. Stearic acid | 2.0 | 2.0 |
| 5. POE (20) stearyl ether | 0.7 | 0.7 |
| 6. Glycyrrhetinic acid | 0.1 | 0.1 |
| 7. Butylparaben | 0.1 | 0.1 |
| 8. Compound I·2Ca | 0.05 | -- |
| 9. Glycerin | 2.0 | 2.0 |
| 10. Carbopol® 941* | 0.1 | 0.1 |
| 11. Ethanol | 10.0 | 10.0 |
| 12. Methylparaben | 0.1 | 0.1 |
| 13. Purified water | balance | balance |
| 14. Perfume | small amount | small amount |

| | | |
|---|---|---|
| *: Carboxyvinyl polymer (molecular weight = 1,000,000 to 1,500,000) | | |

The effectiveness of the milky lotion thus prepared was examined according in the same manner as in Example 4. The results observed are summarized in Table X given below.

**Table X**

| Items Evaluated | | Present Inv. | Comp. Ex. |
|---|---|---|---|
| Improvement of Fine-wrinkles | effective | 2 | 0 |
| | effective in some degree | 13 | 2 |
| | ineffective | 5 | 18 |
| Improvement of Fineness and Glossiness of the Skin | effective | 3 | 0 |
| | effective in some degree | 14 | 3 |
| | ineffective | 3 | 17 |

As seen from the results listed in Table X, the milky lotion of the present invention into which Compound I ·2Ca was incorporated showed marked effects of improving fine-wrinkles and glossiness and fineness of the skin compared with that of Comparative Example free of the compound. In addition, during and after the use of the milky lotion for 3 months, any abnormality such as rubefaction was not observed in the condition of the skin.

Moreover, after the completion of the test, the stability of Compound I ·2Ca in the used milky lotion of the present invention was examined by HPLC and it was found that the compound showed high stability since the rate of Compound I ·2Ca remaining in the milky lotion was 98.8% on the average.

### Example 7

The components 1 to 7 and the components 8 to 12 shown in Table XI were separately mixed and dissolved. To the solution of the components 8 to 12 there was added the solution of the components 1 to 7 with stirring to cause emulsification. The resultant emulsion was cooled to room temperature and the component 13 was added to the emulsion in the course of the cooling to prepare a cream shown in Table XI.

**Table XI**

| Component | Present Inv. | Comp. Ex. |
|---|---|---|
| 1. Liquid paraffin (#70) | 5.0 | 5.0 |
| 2. Squalane | 15.0 | 15.0 |
| 3. Cetostearyl alcohol | 5.0 | 5.0 |
| 4. Beeswax | 2.0 | 2.0 |
| 5. Glyceryl monostearate | 2.0 | 2.0 |
| 6. POE (20) sorbitan monolaurate | 2.0 | 2.0 |
| 7. Propylparaben | 0.1 | 0.1 |
| 8. Compound I·2Zn | 0.5 | -- |
| 9. Hyaluronic acid | 0.2 | 0.2 |
| 10. Diglycerin | 5.0 | 5.0 |
| 11. Methylparaben | 0.2 | 0.2 |
| 12. Purified water | balance | balance |
| 13. Perfume | small amount | small amount |

The effectiveness of the cream thus prepared was examined according in the same manner as in Example 4. The results observed are summarized in Table XII given below.

**Table XII**

| Items Evaluated | | Present Inv. | Comp. Ex. |
|---|---|---|---|
| Improvement of Fine-wrinkles | effective | 6 | 0 |
| | effective in some degree | 11 | 2 |
| | ineffective | 3 | 18 |
| Improvement of Fineness and Glossiness of the Skin | effective | 8 | 0 |
| | effective in some degree | 10 | 2 |
| | ineffective | 2 | 18 |

As seen from the results listed in Table XIl, the cream of the present invention into which Compound I ·2Zn was incorporated showed marked effects of improving fine-wrinkles and glossiness and fineness of the skin compared with that of Comparative Example free of the compound. In addition, during and after the use of the cream for 3 months, any abnormality such as rubefaction was not observed in the condition of the skin.

Moreover, after the completion of the test, the stability of Compound I ·2Zn in the used cream of the present invention was examined by HPLC and it was found that the compound showed high stability since the rate of Compound I ·2Zn remaining in the cream was 99.0% on the average.

### Example 8

Ointments I to IV shown in Table XIII were prepared as agents for treating traumas.

**Table XIII**

| Component | Agents According to the Invention | | | |
|---|---|---|---|---|
| | I | II | III | IV |
| 1. Cetanol | 25.0 | 25.0 | 25.0 | 25.0 |
| 2. White vaseline | 25.0 | 25.0 | 25.0 | 25.0 |
| 3. Propylene glycol | 12.0 | 12.0 | 12.0 | 12.0 |
| 4. POE (40) monostearate | 5.0 | 5.0 | 5.0 | 5.0 |
| 5. Compound I | 0.5 | -- | -- | -- |
| 6. Compound I·6Na | -- | 0.5 | -- | -- |
| 7. Compound I·2Ca | -- | -- | 0.5 | -- |
| 8. Compound I·2Zn | -- | -- | -- | 0.5 |
| 9. Preservative | S.A. | S.A. | S.A. | S.A. |
| 10. Purified water | (balance) | | | |
| Note: The abbreviation "S.A." herein means "small amount". | | | | |

## Claims

1. A composition for external application to skin comprising 0.001 to 5% by weight of CoA-disulfide represented by the following formula (I) and/or a salt thereof selected from the group consisting of alkali metal, alkaline earth metal and zinc family metal salts of the phosphate residue of Compound (I): 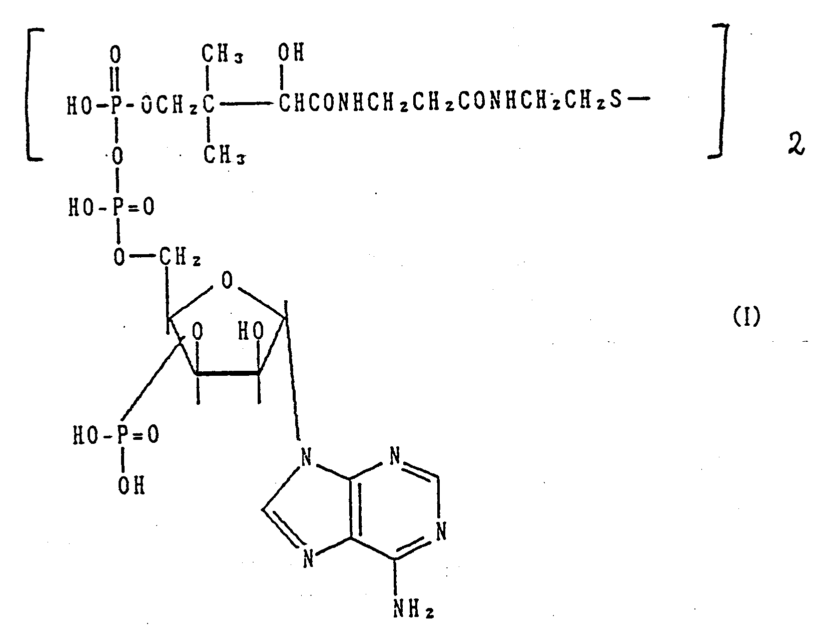 0 to 80% by weight of an oil component, 0 to 12% by weight of a surfactant, 1 to 15% by weight of a humectant, a small amount of a preservative and the balance of an inert carrier.

2. A composition of claim 1 wherein the compound represented by the general formula (I) and/or salt thereof is added to the composition in an amount ranging from 0.01 to 2% by weight on the basis of the total weight of the composition.

3. A composition of claim 1 and/or 2 wherein the composition comprises an inert carrier selected from the group consisting of water, ethanol and isopropanol.

4. A composition of any of claims 1-3 wherein the composition further comprises a surfactant, an oil component, an alcohol, a thickening agent, a preservative, an antioxidant, a chelating agent, a pH adjusting agent, a perfume, a color additive, an ultraviolet absorbing agent, a scattering agent, a vitamin and/or an amino acid.

5. A cosmetic composition for application to skin comprising a composition as set forth in any of claims 1-4.

6. A cosmetic composition of claim 5 wherein it is a cosmetic cream, a milky lotion, a toilet water, a beauty essence, a pack, an undermakeup, a foundation or a jelly.

7. The use of coenzyme A-disulfide represented by the following general formula (I) 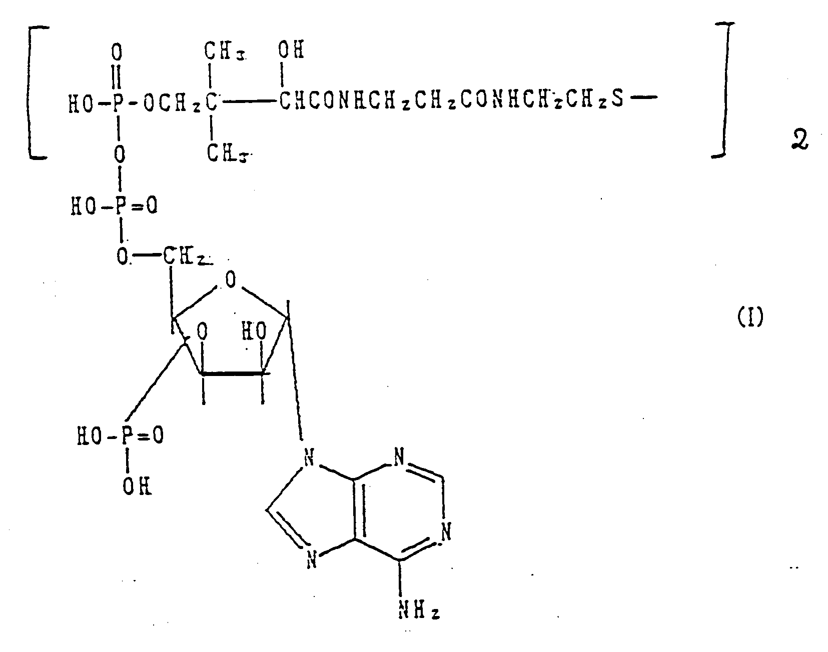 and salts thereof for the preparation of a composition comprising the same for external cosmetic application to skin so as to prevent aging of the skin.

8. The use according to claim 7 wherein the compound represented by the general formula (I) and/or the salt thereof is added to the composition in an amount ranging from 0.001 to 5% by weight on the basis of the total weight of the composition.

9. The use according to claim 7 and/or 8 wherein the compound represented by the general formula (I) and/or the salt thereof is added to the composition in an amount ranging from 0.01 to 2 % by weight on the basis of the total weight of the composition.

10. The use according to any of claims 7-9 wherein the salt compound (I) is selected from the group consisting of alkali metal, alkaline earth metal and zinc family metal salts of the phosphate residue of Compound (I).

11. The use according to any of claims 7-10 wherein the composition comprises an inert carrier selected from the group consisting of water, ethanol and isopropanol.

12. The use according to any of claims 7-11 wherein it comprises 0.001 to 5 % by weight of the compound represented by the general formula (I) and/or salts thereof, 0 to 80 % by weight of an oil component, 0 to 12 % by weight of a surfactant, 1 to 15 % by weight of a humectant, a small amount of a preservative and the balance of an inert carrier.

13. A composition for healing traumas comprising coenzyme A-disulfide represented by the following formula (I) and salts thereof:

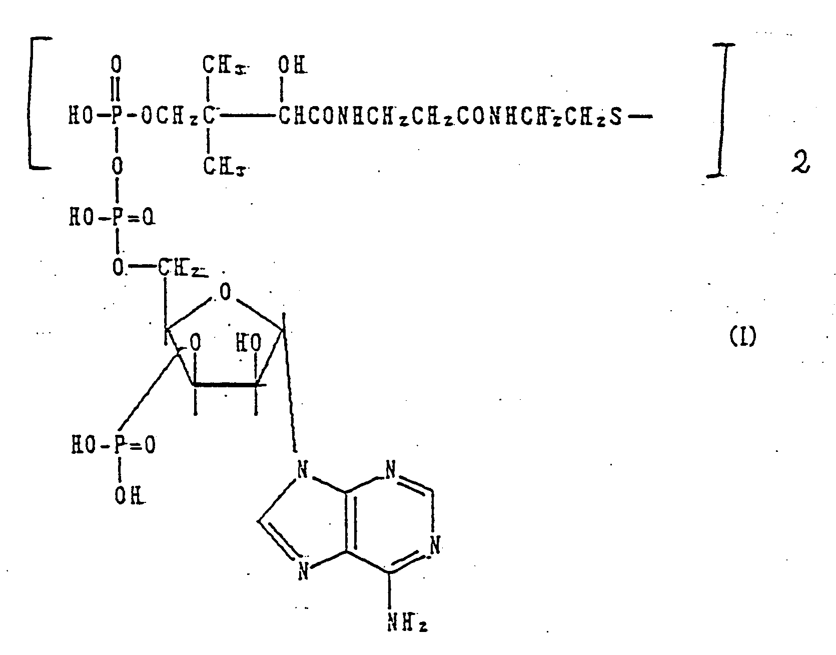

14. A composition of claim 13 wherein the compound represented by the general formula (I) and/or the salt thereof is added to the composition in an amount ranging from 0.001 to 5 % by weight on the basis of the total weight of the composition.

15. A composition of claim 13 wherein it comprises 0.001 to 5 % by weight of the compound represented by the general formula (I) and/or salts thereof, 0 to 80 % by weight of an oil component, 0 to 12 % by weight of a surfactant, 1 to 15 % by weight of a humectant, a small amount of a preservative and the balance of an inert carrier.

## Patentansprüche

1. Präparat zum äußerlichen Gebrauch auf der Haut, enthaltend 0,001 bis 5 Gew.-% CoA-Disulfid der folgenden Formel (I) und/oder eines seiner Salze aus der Gruppe: Alkalimetall-, Erdalkalimetall- und Zinkgruppenmetallsalze des Phosphatrestes der Verbindung (I): 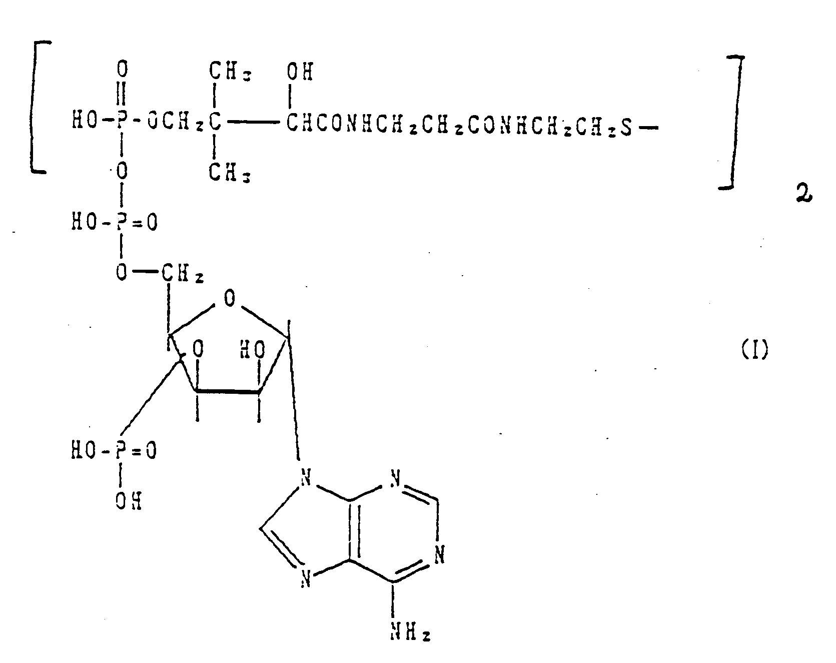 0 bis 80 Gew.-% einer Ölkomponente, 0 bis 12 Gew.-% eines Tensids, 1 bis 15 Gew.-% eines Feuchthaltemittels, eine geringe Menge eines Konservierungsmittels, Rest: inerter Träger.

2. Präparat nach Anspruch 1, wobei die Verbindung der allgemeinen Formel (I) und/oder ihr Salz dem Präparat in einer Menge von 0,01 bis 2 Gew.-%, bezogen auf das Gesamtgewicht des Präparats, zugegeben ist.

3. Präparat nach Anspruch 1 und/oder 2, wobei das Präparat einen inerten Träger aus der Gruppe: Wasser, Ethanol und Isopropanol
umfaßt.

4. Präparat nach einem der Ansprüche 1 bis 3, wobei das Präparat ferner ein Tensid, eine Ölkomponente, einen Alkohol, ein Verdickungsmittel, ein Konservierungsmittel, ein Antioxidans, einen Chelatbildner, ein pH-Einstellungsmittel, ein Parfüm, einen Farbzusatz, ein UV-Absorptionsmittel, ein Zerstreuungsmittel, ein Vitamin und/oder eine Aminosäure umfaßt.

5. Kosmetisches Präparat zur Anwendung auf der Haut, umfassend ein Präparat nach einem der Ansprüche 1-4.

6. Kosmetisches Präparat nach Anspruch 5, das eine kosmetische Creme, eine milchige Lotion, ein Toilette-Wasser, eine Beauty-Essenz, eine Packung, ein Unter-Make-up, eine Grundierung oder ein Gelee ist.

7. Verwendung eines Koenzym A-Disulfids der folgenden allgemeinen Formel (I) 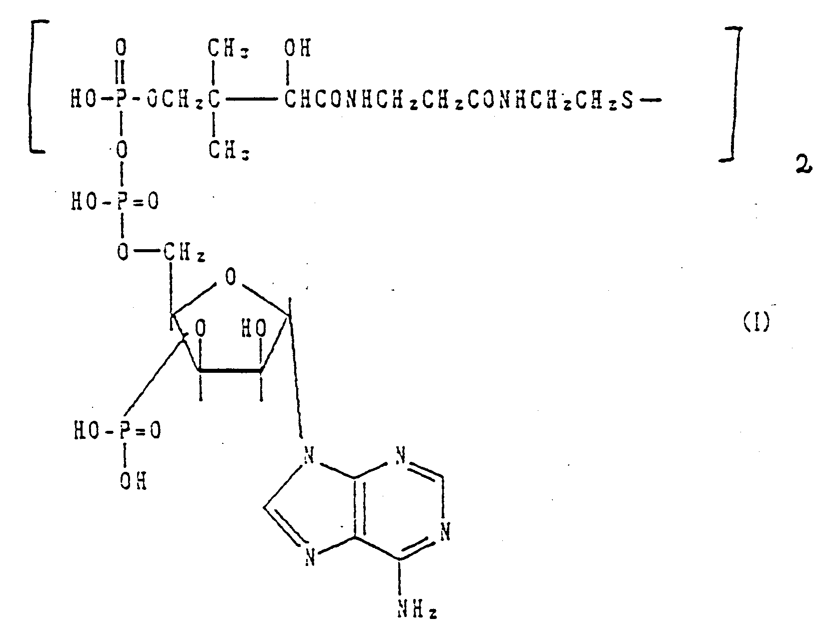 und ihrer Salze zur Herstellung eines dasselbe enthaltenden Präparats zur äußerlichen kosmetischen Anwendung auf die Haut zur Verhinderung der Hautalterung.

8. Die Verwendung nach Anspruch 7, wobei die Verbindung der allgeinen Formel (I) und/oder ihr Salz dem Präparat in einer Menge von 0.001 bis 5 Gew.-%, bezogen auf das Gesamtgewicht des Präparats, zugesetzt ist.

9. Verwendung nach Anspruch 7 und/oder 8, wobei die Verbindung der allgemeinen Formel (I) und/oder ihr Salz dem Präparat in einer Menge von 0,01 bis 2 Gew.-%, bezogen auf das Gesamtgewicht des Präparats, zugegeben ist.

10. Verwendung nach einem der Ansprüche 7 bis 9, wobei die Salzverbindung (I) aus der Gruppe: Alkalimetall-, Erdalkalimetall- und Zinkgruppenmetallsalze des Phosphatrests der Verbindung (I) ausgewählt ist.

11. Verwendung nach einem der Ansprüche 7 bis 10, wobei das Präparat einen inerten Träger aus der Gruppe: Wasser, Ethanol und Isopropanol
enthält.

12. Verwendung nach einem der Ansprüche 7 bis 11, wobei es 0,001 bis 5 Gew.-% der Verbindung der allgemeinen Formel (I) und/oder ihrer Salze, 0 bis 80 Gew.-% einer Ölkomponente, 0 bis 12 Gew.-% eines Tensids, 1 bis 15 Gew.-% eines Feuchthaltemmittels, eine kleine Menge eines Konservierungsmittels, Rest: inerter Träger enthält.

13. Präparat für die Wundheilung, enthaltend Koenzym A-Disulfid der folgenden Formel (I) und ihre Salze:

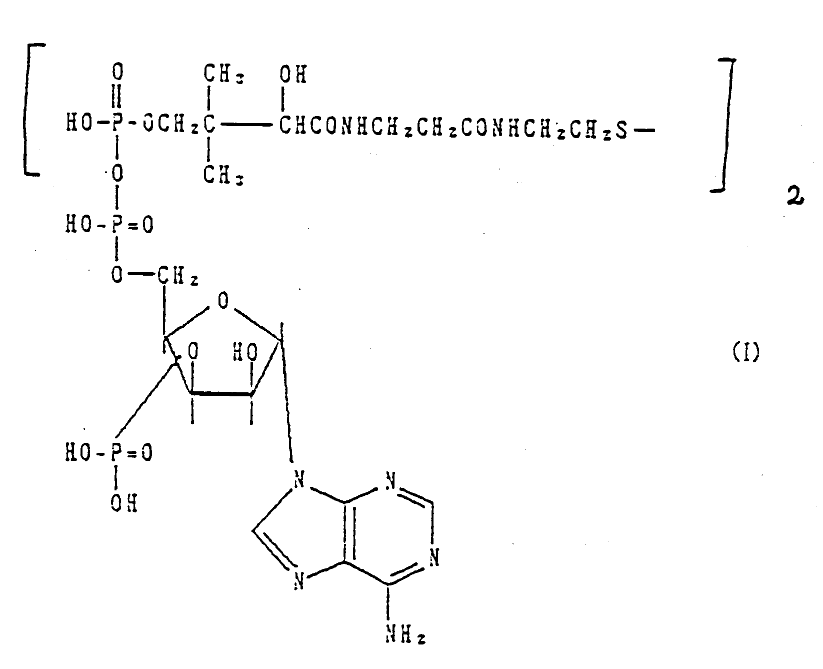

14. Präparat nach Anspruch 13, wobei die Verbindung der allgemeinen Formel (I) und/oder ihr Salz dem Präparat in einer Menge von 0,001 bis 5 Gew.-%, bezogen auf das Gesamtgewicht des Präparats, zugegeben ist.

15. Präparat nach Anspruch 13, wobei es 0,001 bis 5 Gew.-% der Verbindung der allgemeinen Formel (I) und/oder ihrer Salze, 0 bis 8 Gew.-% einer Ölkomponente, 0 bis 12 Gew.-% eines Tensids, 1 bis 15 Gew.-% eines Feuchthaltemittels, eine geringe Menge Konservierungsmittel, Rest: ein inerter Träger
enthält.

## Revendications

1. Une composition pour l'application externe sur la peau comprenant 0,001 à 5 % en poids de disulfure de CoA représenté par la formule suivante (I) et/ou un sel de ce composé choisi dans le groupe comprenant les sels de métaux alcalins, de métaux alcalino-terreux et de métaux de la famille du zinc du résidu phosphate du composé (I) : 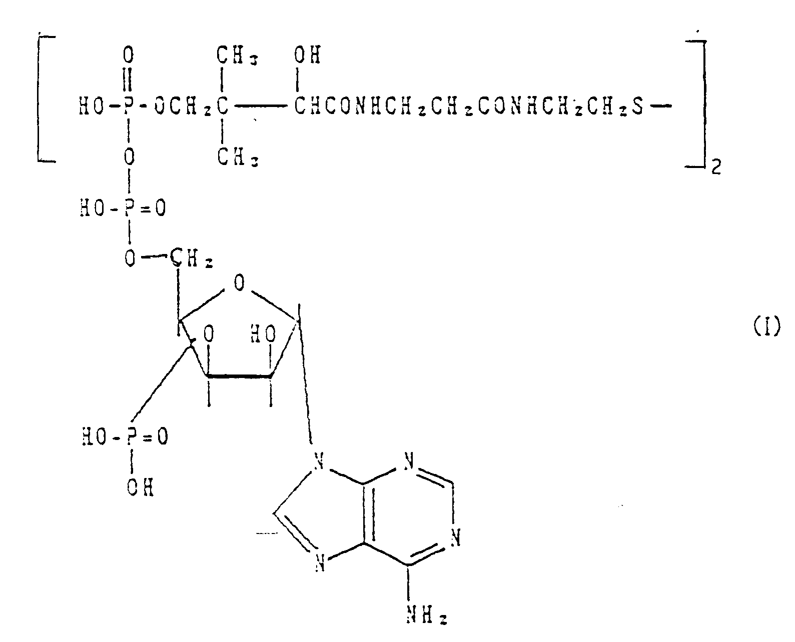 0 à 80 % en poids d'une huile, 0 à 12 % en poids d'un agent surfactant, 1 à 15 % en poids d'un agent humectant, une faible quantité de conservateur et le restant étant un support inerte.

2. Une composition selon la revendication 1, selon laquelle le composé représenté par la formule générale (I) et/ou un sel de ce composé est ajouté à la composition en quantité dans l'intervalle de 0,01 à 2 % en poids sur la base du poids total de la composition.

3. Une composition selon la revendication 1 et/ou 2, selon laquelle la composition comprend un support inerte choisi dans le groupe comprenant l'eau, l'éthanol et l'isopropanol.

4. Une composition selon l'une quelconque des revendications 1 à 3, selon laquelle la composition comprend également un agent surfactant, une huile, un alcool, un agent épaississant, un conservateur, un antioxydant, un agent chélatant, un agent d'ajustement du pH, un parfum, un additif colorant, un agent absorbant l'ultraviolet, un agent dispersant, une vitamine et/ou un aminoacide.

5. Une composition cosmétique pour l'application à la peau comprenant une composition selon l'une quelconque des revendications 1 à 4.

6. Une composition cosmétique selon la revendication 5, selon laquelle il s'agit d'une crème cosmétique, d'une lotion de lait, d'une eau de toilette, d'une essence de beauté, d'un concentré, d'un sous-maquillage, d'un fond de maquillage ou d'une gelée.

7. L'utilisation du disulfure de coenzyme A représenté par la formule générale suivante (I) : 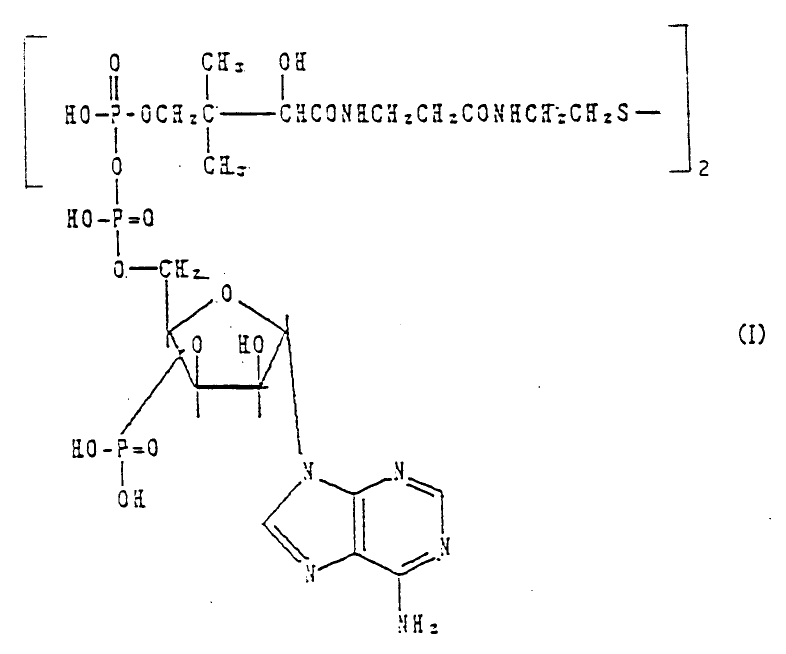 et les sels de celui-ci pour la préparation d'une composition comprenant le même pour l'application cosmétique externe à la peau afin de prévenir le vieillissement de la peau.

8. L'utilisation selon la revendication 7, selon laquelle le composé représenté par la formule générale (I) et/ou un sel de ce dernier est ajouté à la composition en quantité dans l'intervalle de 0,001 à 5 % en poids sur la base du poids total de la composition.

9. L'utilisation selon la revendication 7 et/ou 8, selon laquelle le composé représenté par la formule générale (I) et/ou le sel de ce dernier est ajouté à la composition en quantité dans l'intervalle de 0,01 à 2 % en poids sur la base du poids total de la composition.

10. L'utilisation selon l'une quelconque des revendications 7-9, selon laquelle le sel du composé (I) est choisi dans le groupe comprenant les sels de métaux alcalins, les sels de métaux alcalino-terreux et les sels de métaux de la famille du zinc du résidu phosphate du composé (I).

11. L'utilisation selon l'une quelconque des revendications 7-10, selon laquelle la composition comprend un support inerte choisi dans le groupe comprenant l'eau, l'éthanol et l'isopropanol.

12. L'utilisation selon l'une quelconque des revendications 7-11, selon laquelle elle comprend 0,001 à 5 % en poids du composé représenté par la formule générale (I) et/ou des sels de ce dernier, 0 à 80 % en poids d'une huile, 0 à 12 % en poids d'un agent surfactant, 1 à 15 % en poids d'un agent humectant, une faible quantité de conservateur et le restant étant un support inerte.

13. Une composition pour guérir les traumas comprenant le disulfure de coenzyme A représenté par la formule générale (I) et ses sels :

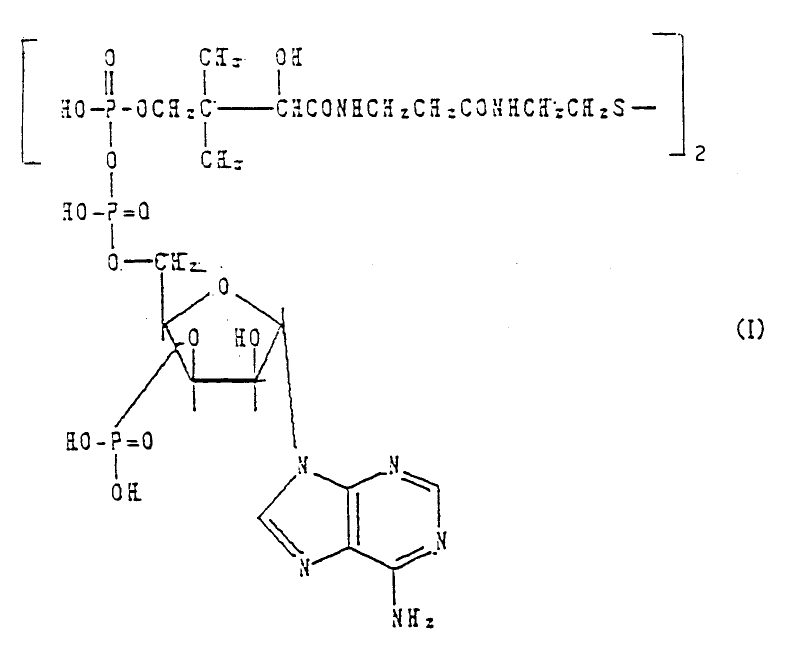

14. Une composition selon la revendication 13, selon laquelle le composé représenté par la formule générale (I) et/ou un sel de ce dernier est ajouté à la composition en quantité dans l'intervalle de 0,001 à 5 % en poids sur la base du poids total de la composition.

15. Une composition selon la revendication 13, selon laquelle elle comprend 0,001 à 5 % en poids du composé représenté par la formule générale (I) et/ou des sels de ce composé, 0 à 80 % en poids d'une huile, 0 à 12 % en poids d'un agent surfactant, 1 à 15 % en poids d'un agent humectant, une faible quantité d'un conservateur et le restant étant un support inerte.
